Europäisches Patentamt

European Patent Office

Office européen des brevets

(11) Numéro de publication: **0 158 546**
**B1**

# FASCICULE DE BREVET EUROPEEN

(12)

(45) Date de publication du fascicule du brevet:
21.02.90

(21) Numéro de dépôt: 85400446.2

(22) Date de dépôt: 07.03.85

(51) Int. Cl.⁵: **C 12 N 15/00, C 12 N 5/00,**
C 12 P 21/00 // A61K37/02,
A61K39/29 ,(C12P21/00,
C12R1:91)

(54) Cellules hybrides productrices d'un polypeptide déterminé et obtenu à partir de cellules primaires naturellement capables d'exprimer ce polypeptide ou un polypeptide équivalent, procédé pour l'obtention de ces cellules hybrides et application de celles-ci à la production dudit polypeptide.

(30) Priorité: 07.03.84 FR 8403565

(43) Date de publication de la demande:
16.10.85 Bulletin 85/42

(45) Mention de la délivrance du brevet:
21.02.90 Bulletin 90/8

(84) Etats contractants désignés:
AT BE CH DE GB IT LI LU NL SE

(56) Documents cités:
EP-A- 0 022 685
EP-A- 0 093 436
EP-A- 0 093 436
WO-A-82/03087
FR-A- 2 487 852
GB-A- 2 010 847

(73) Titulaire: **CENTRE NATIONAL DE LA RECHERCHE
SCIENTIFIQUE (CNRS), 15, Quai Anatole France,
F-75007 Paris (FR)**
Titulaire: **INSTITUT PASTEUR, 25-28, rue du Docteur
Roux, F-75724 Paris Cédex 15 (FR)**

(72) Inventeur: **Chenciner, Nicole, 11 Quai Bourbon,
F-75004 Paris (FR)**
Inventeur: **Houssais, Jean-François, 35 Allée des
Comtes de Montford-Auffargis, F-78610 Le Perray en
Yvelines (FR)**

(74) Mandataire: **Gutmann, Ernest et al, S.C. Ernest Gutmann
- Yves Plasseraud 67, boulevard Haussmann,
F-75008 Paris (FR)**

ACTORUM AG

## Description

L'invention est relative à des hybrides cellulaires efficaces pour la production d'un polypeptide déterminé codé par une séquence d'ADN cloné ou clonable dans cet hybride cellulaire, à un procédé de production de ces hybrides cellulaires et à l'application de ces derniers à la production dudit polypeptide.

La production d'hybrides cellulaires a déjà été envisagée, notamment dans des études sur les expressions des fonctions constitutives et différenciées des cellules à partir desquelles ils avaient été obtenus. Par exemple Bertolotti et al ((1973), J. Cell. Physiol. 79, 211–224) ont réalisé des hybrides cellulaires, par fusion de cellules d'hépatome de rat avec des fibroblastes de souris ou des cellules épithéliales de rat, en vue de l'étude de l'expression ou de la non-expression de certaines des fonctions spécifiques exprimées par les cellules initiales. Ces auteurs devaient constater que la capacité des cellules d'hépatome de rat de produire l'aldolase était atteinte dans l'hybide formé.

D'autres auteurs, par exemple KLEBE et al ((1970), Proc. Natl. Acad. Sci. USA 66, 1220–1227) ont également étudié les capacités de cellules hybrides à exprimer ou à réexprimer des fonctions spécifiques propres à l'un au moins des constituants cellulaires mis en jeu dans la formation de l'hybride.

Ces auteurs ont notamment observé que dans les hybrides entre des cellules d'adénocarcinome rénal de souris produisant l'estérase ES2 et des fibroblastes diploïdes humains, ES2 n'était pas produit si les chromosomes humains étaient maintenus, mais que la perte de ceux-ci entraînait une réexpression de ES2. On mentionnera également des études analogues de FOUGERE et al (1972), P.N.A.S. 69, 330–334, qui rapportèrent la capacité de certains clones hybrides entre des cellules de mélanome de hamster Syrien et de fibroblastes de souris d'exprimer la mélanine avec un taux de production accru. Celle-ci a pu être corrélée à la présence dans l'hybride, révélée par leur caryotype de deux génomes de cellules mélanome et d'un génome de fibroblaste de souris.

La technique décrite dans la demande EP-A-93346 concerne un procédé d'immortalisation de cellules primaires, au sens qui sera donné ci-après de cette expression, avec des fragments originaires de lignées cellulaires immortalisées en l'absence de marqueur de sélection, cette dernière étant opérée par la récupération de celles des cellules qui se multiplient dans les conditions de culture utilisées.

La présente invention découle d'une recherche mettant également en jeu des hybrides cellulaires, recherche néanmoins toute différente, dont la démarche a essentiellement été de combiner les avantages des techniques du génie génétique et de la production des hybrides cellulaires. Il est bien connu à ce jour que l'on peut transformer des micro-organismes par des séquences d'ADN clonées, dans des conditions les rendant propres à synthétiser une protéine ou un polypeptide étranger. Cette production imposée à ces micro-organismes s'effectue cependant dans un environnement génétique qui n'est pas toujours favorable, de sorte que le rendement de production de cette protéine ou de ce polypeptide, ou même simplement les conditions de survie ou de développement des micro-organismes concernées, peuvent s'en trouver considérablement affectés.

L'invention a pour but de fournir des micro-organismes modifiés transformés ou transformables par des séquences d'ADN clonées par la mise en œuvre des techniques du génie génétique, ces micro-organismes modifiés étant cependant tels que la séquence d'ADN clonée se trouve en fait replacé dans un environnement génétique (intervenant par exemple au niveau de la différenciation ou de la programmation des génomes contenant une séquence d'ADN identique ou équivalente) proche des conditions naturelles qui s'avèrent en général particulièrement favorable à son expression.

Le but de l'invention est encore de fournir des hybrides cellulaires capables d'exprimer une séquence d'ADN clonée, avec des rendements de production en polypeptide correspondant, rarement sinon jamais atteints, dans les micro-organismes rendus producteurs d'un tel polypeptide grâce aux techniques du génie génétique.

Le procédé selon l'invention d'un hybride cellulaire exprimant ou susceptible d'être rendu capable d'exprimer une séquence d'ADN cloné déterminée est caractérisé:

– en ce que l'on réalise une coculture, dans des conditions propres à permettre leur hybridation, d'une part, de cellules eucaryotes établies, dans lesquelles la susdite séquence d'ADN clonée est exprimable ou exprimée et, d'autre part, de cellules primaires naturellement favorables à l'expression de gènes naturels codant pour une protéine constituée par le polypeptide codé par la susdite séquence d'ADN clonée ou contenant dans sa propre structure une séquence polypeptidique identique ou analogue à celle de ce polypeptide, cette coculture étant réalisée dans un milieu qui n'autorise pas le développement desdites cellules établies, lorsqu'elles ne sont pas complémentées par une séquence génétique appropriée,

– en ce que les cellules primaires mises en jeu dans l'hybridation cellulaire sont aptes ou ont été rendues aptes au préalable à fournir ladite séquence génétique appropriée à au moins une partie des hybrides cellulaires formés dans ledit milieu de culture et en ce que l'on recueille les hybrides cellulaires formés.

Le procédé selon l'invention peut permettre l'obtention d'hybrides cellulaires directement producteurs du polypeptide déterminé recherché si, soit les cellules établies, soit les cellules primaires, voire même les deux à la fois, avaient été transformées préalablement à la coculture susdite avec la susdite séquence d'ADN clonée. Dans un tel cas, le procédé selon l'invention pourra comprendre l'étape supplémentaire que constitue la sélection, après ladite coculture de ceux desdits hybrides cellulaires formés, qui se révèlent aussi être producteurs du polypeptide codé par le susdit ADN cloné.

En variante, les hybrides cellulaires obtenus à l'issue de la susdite coculture des cellules eucaryotes établies et des cellules primaires naturellement favorables, respectivement non transformées, peuvent être eux-mêmes transformés par la susdite séquence d'ADN clonée, le procédé selon l'invention devant alors, dans ce mode de mise en œuvre préféré, comprendre la sélection après ladite coculture de ceux des hybrides cellulaires qui ont ainsi été rendus producteurs du polypeptide codé par le susdit ADN cloné.

La compréhension des définitions qui précèdent se trouvera facilitée par l'exposé immédiat, à titre d'exemple, des conditions d'obtention d'hybrides cellulaires préférés capables ou susceptibles d'être rendus capables de produire des quantités importantes d'un polypeptide vaccinant contre l'hépatite virale B.

Dans le cadre de cette application préférée, le procédé selon l'invention peut être défini comme un procédé de fabrication d'un hybride cellulaire exprimant ou susceptible d'être rendu capable d'exprimer une séquence d'ADN clonée contenant tout ou partie d'un gène déterminé contenu dans le génome du virus de l'hépatite B ou codant pour un peptide immunogène ayant des propriétés immunologiques équivalentes à celles du polypeptide codé par ce gène, ce procédé étant caractérisé:

– en ce que l'on réalise une coculture, dans des conditions propres à permettre leur hybridation, d'une part, de cellules eucaryotes établies dans lesquelles la susdite séquence d'ADN clonée est exprimable ou exprimée et, d'autre part, d'hépatocytes, de préférence humains, cette coculture étant réalisée dans un milieu qui n'autorise pas le développement desdites cellules établies, lorsque celles-ci ne sont pas complémentées par une séquence génétique appropriée,

– en ce que les hépatocytes mis en jeu dans l'hybridation cellulaire, sont aptes ou ont au préalable été rendus aptes à fournir ladite séquence génétique appropriée à au moins une partie des hybrides cellulaires formés dans ledit milieu de culture et en ce que l'on recueille les hybrides cellulaires formés.

Selon une première alternative de ce procédé préféré, soit les cellules établies, soit les hépatocytes, soit les deux à la fois, avaient été transformés préalablement à la coculture susdite avec la susdite séquence d'ADN clonée. Le procédé selon l'invention comprend alors une sélection, après la susdite coculture, des susdits hybrides cellulaires formés qui se révèlent aussi producteurs du peptide immunogène ou de l'antigène codé par la susdite séquence d'ADN clonée. En variante, ce sont les hybrides cellulaires obtenus, bien que non secréteurs, qui sont transformés par la susdite séquence d'ADN, le procédé selon l'invention comprenant alors encore la sélection, après la susdite coculture, de ceux des hybrides cellulaires formés qui se révèlent aussi être producteurs de l'antigène ou du polypeptide immunogène codé par la séquence d'ADN déterminée.

Dans ce qui précède, l'hybride non secréteur pouvait aussi bien être constitué par l'hybride obtenu à partir de cellules primaires et de cellules établies qui n'avaient pas elles-mêmes été transformées au préalable. Celles-ci pouvaient cependant l'avoir également été. Dans ce cas, l'hybride utilisé pour la transformation pouvait être l'un de ceux qui n'avait pas été retenu dans le cadre de la sélection des hybrides cellulaires envisagée dans la variante précédente.

L'expression «cellules primaires» telle qu'utilisée plus haut désigne toute cellule prélevée sur un mammifère et connue par sa capacité à être le siège de la production de la protéine ou du polypeptide correspondant à cette protéine dont la production est recherchée. Ces cellules primaires sont caractérisées par le fait qu'elles ne se multiplient guère in vitro, que leur prolifération est tout au plus limitée à la production de monocouches, le développement étant ensuite interrompu par «inhibition de contact» ou analogue. Ceci étant, ces cellules peuvent tout de même, en tout état de cause, être maintenues un certain temps en survie dans des milieux appropriés. Ces cellules primaires sont notamment constituées par des hépatocytes, plus particulièrement des hépatocytes humains, dans le cas préféré qui a été évoqué plus haut. Les cellules primaires utilisées pour la mise en œuvre du procédé selon l'invention seront toujours choisies en fonction de leur capacité à exprimer dans la nature une séquence génique codant pour la protéine ou le polypeptide recherché. Il peut s'agir de cellules primaires qui spontanément produisent la protéine ou le polypeptide du genre en question, ou encore de cellules primaires dont est connue la capacité de transformation naturelle, par exemple par un virus, d'exprimer le gène en question. A cet égard, et toujours encore dans le cas de l'exemple préféré sus-indiqué, les hépatocytes, notamment humains, sont particulièrement préférés pour l'expression des séquences d'ADN issues du génome du virus de l'hépatite B (DNA-HBV) ou de séquences codant pour les mêmes polypeptides. En effet, le hépatocytes, notamment humains ou de certaines lignées de chimpanzés, constituent des cibles préférées du virus de l'hépatite B.

On pourrait de la même façon utiliser à titre de cellules primaires des cellules d'hypophyse lorsque la protéine dont la synthèse est recherchée est constituée par l'hormone de croissance ou de lignées de lymphocytes lorsque la protéine est constituée par l'interféron lymphocytaire.

L'expression «cellules eucaryotes établies» désigne des cellules eucaryotes, plus particulièrement de mammifères qui peuvent être cultivées in vitro et qui sont capables de se multiplier à travers plusieurs générations.

Tout type de cellules établies, également dérivées de cellules de mammifères, peut être utilisé. Il s'agit par exemple de cellules tumorales ou non, de mutants de cellules naturelles ou primaires, mais auxquelles la mutation a conféré la capacité de multiplication propre aux cellules établies. Ces mutants peuvent être naturels ou induits. Il s'agit encore de toutes catégories de cellules pouvant être maintenue en culture, telles que des lignées cellulaires dépourvues de caractère oncogène, comme par exemple des lignées cellulaires hétéroploïdes Vero de primates non humains, telles que celles décrites par Petricciani, J.C., Kirschstein, R.L., Hines, J.E., Wallace R.E. et Martin, D.P. (1973), «J. Natl. Cancer Inst.», 51, 191-196, ces dernières cellules s'étant révélées non tumorigènes, même lorsque testées dans des singes soumis à un traitement immunosuppresseur. On peut encore mentionner des lignées cellulaires Hela humaines ou des singes.

Le procédé selon l'invention a donc par objet de réunir dans un même organisme l'environnement génétique le plus favorable à la production de la protéine ou du polypeptide recherché, et en tous les cas aussi proche que possible de l'environnement génétique naturel, d'une part, et la capacité de multiplication sur plusieurs générations des lignées cellulaires cultivables in vitro, d'autre part.

Le milieu de culture, aussi bien que les cellules établies mises en œuvre, sont choisis de façon à satisfaire aux conditions qui ont été définies plus haut. Le milieu est tel et les cellules établies sont telles que celles-ci ne peuvent pas se développer dans le milieu choisi, sauf à être complémentées par une séquence génétique appropriée, susceptible d'être apportée par les cellules primaires avec lesquelles elles sont cocultivées.

Les cellules établies sont par exemple constituées par des cellules présentant une déficience naturelle ou induites au niveau d'un de leurs gènes, cette déficience leur interdisant de se développer dans un milieu déterminé, cette déficience pouvant cependant être compensée par l'incorporation d'un gène homologue exprimable sous la forme d'une protéine homologue à celle qui aurait été spécifiée par le gène correspondant, s'il n'avait été déficient. Les cellules ayant incorporé le gène homologue sont alors à même de se développer dans le milieu considéré. C'est à cette possibilité de compensation que se réfère l'expression «complémentation» qui a été utilisée dans la définition la plus générale indiquée plus haut du procédé selon l'invention. Divers exemples de gènes ou de séquences d'ADN complémentables ont été mentionnés par exemple dans la demande de brevet France n° 81 01137 déposée le 2 mars 1981. A ce type d'ADN appartiennent notamment ceux du virus dit «Herpès simplex Virus» du type 1 (HSVI), le gène de la thymidine-kinase (TK), le gène de l'adénine phosphoribosyl-transférase (APRT) et de la dihydrofolate réductase (DHFR), etc. de l'enzyme xanthineguanine phosphoribosyl-transférase (XGPRT), ou de l'hypoxanthine guanine phosphoribosyl transférase (HGPRT).

A chacun de ces «ADN de complémentation» correspondant des milieux répondant aux conditions sus-indiquées. Partant par exemple de cellules établies constituées par des cellules de souris du type L dont les gènes codant pour la thymidine-kinase endogène sont affectés par une mutation induite ou provoquée (cellules L-TK-) ou par des cellules HGPRT-, on aura avantageusement recours au milieu connu sous le nom de «milieu HAT» (contenant de l'hypoxanthine et de l'amino-ptérine en sus de la thymidine). Ce milieu est connu pour n'autoriser l'éventuelle synthèse de thymidine phosphate que par l'intermédiaire d'une voie métabolique dite «voie de sauvetage» (salvatior pathway), cette voie impliquant cependant que soit préservée l'intégrité du gène TK des cellules susceptibles de s'y développer. Dans la mesure où le gène ou la séquence nucléique de complémentation leur est apporté par les cellules primaires (par exemple cellules primaires TK+ ou HGPRT+), on conçoit que seuls les hybrides auxquels ont été transmis le gène homologue susdit au cours de la fusion cellulaire peuvent se développer dans le milieu de culture choisi. Les cellules établies non engagées dans la constitution de l'hybride et non complémentées ne peuvent s'y développer, tout comme les cellules primaires elles-mêmes qui, par nature, n'ont qu'une durée de survie limitée.

L'ADN de complémentation peut être constitué par une séquence naturelle, appartenant au génome de la cellule primaire. Il peut éventuellement avoir été fourni au préalable aux cellules primaires, dans la mesure où celles-ci en étaient initialement dépourvues, notamment par transformation préalable avec la séquence d'ADN appropriée.

Dans un mode de mise en œuvre équivalent du procédé selon l'invention, on aura recours à l'introduction dans le milieu d'une substance susceptible d'interdire la prolifération, voire de tuer rapidement les deux types de cellules, dans la mesure où à cette substance correspond une deuxième substance, notamment une enzyme, susceptible d'inactiver la première, la seconde étant codée par une séquence d'ADN déterminée, susceptible d'être introduite préalablement dans les cellules primaires. La première substance est par exemple constituée par un antibiotique, tel que celui connu sous la désignation G418, la seconde substance étant alors constituée par un aminoglycoside 3'-phosphotransférase. On pourra encore se référer pour l'illustration de ce principe à la demande de brevet déjà mentionnée plus haut n° 81 0437. Comme dans le cas précédent, seul survivra l'hybride, qui aura «reçu en héritage» de la cellule primaire la séquence d'ADN codant pour ladite aminoglycoside 3'-phosphotransférase.

La séquence d'ADN clonée, dont l'expression est recherchée, peut être constituée par tout fragment de gène naturel ou toute séquence résultant d'une transcription réverse d'un brin d'acide nucléique monocaténaire, par exemple d'un mARN, par voie enzymatique, voire par tout ADN obtenu par synthèse chimique. Par exemple, cette séquence est constituée par un cADN codant pour un polypeptide identique à celui exprimé par le gène naturel correspondant ou un polypeptide équivalent, l'équivalence résultant notamment de l'analogie immunologique susceptible d'être reconnue entre ces deux polypeptides dans des réactions croisées correspondantes avec des anticorps préalablement formés contre le polypeptide naturel et le polypeptide équivalent. Quelle que soit la séquence d'ADN utilisée, le choix des lignées de cellules primaires sera fait en fonction du gène naturel correspondant.

Pour réaliser la transformation ou la transfection des cellules établies ou des cellules primaires, avant hybridation de celles-ci, soit encore des hybrides préalablement formés, on peut avoir recours à tout vecteur, notamment plasmide approprié. Les conditions auxquelles ce vecteur doit satisfaire sont uniquement qu'elles permettent la transformation effective et l'expression de la séquence d'ADN clonée dans les cellules utilisées. L'homme du métier sera parfaitement à même de choisir les types de plasmide à mettre en œuvre, compte tenu de la nature des cellules à mettre en œuvre dans l'hybridation et le cas échéant de la séquence d'ADN à cloner. Lorsque la séquence à cloner ne se trouve pas déjà placée sous le contrôle du pormoteur qui permettra l'expression de cette séquence au sein de la cellule transformée, il sera avantageux de la mettre sous le contrôle direct d'un promoteur choisi parmi ceux qui permettent non seulement l'expression, mais l'excrétion du polypeptide exprimé dans le milieu de culture. Des promoteurs avantageux seront par exemple ceux sous le contrôle desquels se trouvent les gènes S et les séquences pré-S de DNA-HBV, ou des promoteurs forts tels que ceux obtenus à partir du virus SV40 ou du virus MMTV.

Bien entendu, les vecteurs en question devront encore comprendre tous les éléments génétiques susceptibles d'assurer la transcription et la traduction de la séquence d'ADN clonée dans une cellule eucaryote, plus particulièrement de mammifère, par exemple les sites de polyadénylation, et de préférence aussi de la terminaison de la transcription de la séquence d'ADN concernée. Ceci étant, il n'est pas nécessaire d'insister sur la constitution des plasmides susceptibles d'être utilisés. Un grand nombre de ces plasmides ont été décrits dans la littérature technique. Ils peuvent tous être mis en œuvre dès lors qu'ils sont reconnus capables de transformer des cellules de mammifère et d'apporter à ces dernières le gène ou la séquence d'ADN clonée susceptible d'être transcrite et exprimée dans ces cellules.

Enfin l'hybridation proprement dite des cellules primaires et établies peut être réalisée dans des conditions classiques. Elle met en œuvre de façon en soi connue tout agent capable de fragiliser les parois cellulaires, au point de rendre la fusion possible. On peut classiquement utiliser le virus de Sendaï ou plus avantageusement encore des polyalcoylène-polyols (PEG 1000), notamment le polyéthylène-glycol, par exemple à raison de 500 mg/ml ou à raison d'une solution de 40% poids/volume de milieu de culture sans sérum du milieu de culture pendant 1 minute.

D'autres caractéristiques préférées de l'invention apparaîtront encore au cours de la description de modes de mise en œuvre préférés du procédé selon l'invention dans les exemples qui suivent.

Exemple I

Des lignées cellulaires de fibroblastes de souris (cellules L TK-) produisant l'antigène HBs après transfection du gène HBs du virus HBV ont été fusionnées avec des hépatocytes de rat en culture primaire. Avec l'une des lignées de départ, il a été possible d'obtenir des clones hybrides dont le taux de production de l'antigène HBs a été multiplié par plus de 20.

Les techniques d'obtention des clones hybrides secréteurs et les résultats obtenus sont décrits dans les exemples qui suivent.

1. Origine des plasmides contenant les ADN clonés et utilisés pour des ADN transfections.

Le plasmide pACF10 est dérivé du plasmide pAC2 décrit par Pourcel et al (1982), J. of Virology, 42, 100, portant le gène codant pour la région pré-S et S du virus HBV. Le plasmide pACF10 se distingue de pAC2 par l'introduction dans le site HindIII de ce plasmide du fragment BamHI-EcoRI (3918–1656) de l'ADN du virus polyome codant pour une partie des protéines précoces et par la délétion du gène codant pour la thymidine kinase. Une souche de E. coli K12 transformée par pACF10 a été déposée à C.N.C.M. le 5 mars 1984 sous le n° I-284.

ADN du plasmide pNY4. Ce plasmide porte le fragment BglII de DNA-HBV contenant la séquence d'ADN codant pour la région pré-S et S placée derrière le fragment Hae du virus polyome (fragment portant l'origine de réplication du virus polyome et le promoteur précoce), le fragment de DNA-HBV ayant été inséré par génie génétique dans le site HaeII dudit promoteur précoce.

ADN du plasmide pW. Ce plasmide porte le gène codant pour l'aminoglycoside-3'-phosphotransférase conférant la résistance à l'aminoglycoside G418 aux cellules l'exprimant (Colbère-Garapin et Garapin, 1981 et brevet n° 81 0437).

2. Obtention des lignées cellulaires de souris produisant la protéine HBs, après transfection puis sélection des clones producteurs.

Des cellules LMTK- (clone 1D) (cellules de souris dérivées du clone L929 déficientes en thymidine kinase, poussant dans du milieu de Eagle modifié par Dulbecco, supplémenté par 10% de sérum de veau et déposées le 5 mars 1984 à la Collection Nationale des Cultures de Micro-organismes de l'Institut Pasteur de Paris (C.N.C.M.) sous le n° I-283) ont été cotransfectées selon la technique décrite par Graham, F.L. et Van der Eb, A.J. (1973), Virology 52, p. 456, par de l'ADN de deux vecteurs, l'un portant la région codante pour la protéine HBs (plasmide pACF10 ou plasmide pNY4 sous-type ayw), l'autre (plasmide pW) portant le gène de l'aminoglycosi de 3'-phosphotransférase (APH 3'). Les cellules transfectées exprimant l'enzyme APH 3' sont sélectionnées en présence de 400 microG/ml d'aminoglycoside G418, selon la méthode de Colbère-Garapin décrite dans l'article mentionné plus haut. Les clones issus de cette sélection sont alors testés pour la production de la protéine HBs. 5.10⁵ cellules L TK- ont été cotransfectées par 10 microgrammes du plasmide pACF10 ou pNY4 et par 2 microgrammes d'ADN du plasmide pW. Quatre jours après la transfection, le milieu sélectif était appliqué.

Trois lignées cellulaires (L1, L2, L3), obtenues après cotransfection des cellules LMTK-, et dont avait été reconnue la capacité à produire un antigène correspondant à l'antigène HBsAg de l'enveloppe du virus de l'hépatite B, ont été prélevées au hasard et utilisées pour les essais de fusion avec les hépatocytes de rat (H A). La présence d'AgHBs fut détectée à l'aide des tests radio-immunologiques AUS-RIAII (Abbott). La quantité d'AgHBs excrétée dans le milieu fut déterminée par rapport à une courbe étalon établie par dilution d'un standard d'AgHBs de l'Institut Pasteur Production.

L1 et L3 étaient dérivées de cellules LTK- transfectées par l'ADN du plasmide PACF10 et L2 de cellules transfectées par l'ADN du plasmide pNY4.

La lignée L1 a été déposée le 5.03.84 à la C.N.C.M. sous le n° I-281.

Les niveaux de production de l'antigène HBs dans ces trois lignées étaient les suivantes:

L1 50–75 ng

L2 60 ng par 10⁶ cellules et par 24 heures

L3 68 ng.

3. Mise en culture des hépatocytes de rat en culture primaire.

Des hépatocytes de rat Wistar ont été isolés après perfusion du foie par la collagénase, selon la méthode de Seglen (1973), avec quelques modifications, Guguen-Guillouzo et al, 1980, In vitro 16, J.F. Houssais et al, 1983, C.R. Acad. Sci. Paris, tome 297, série III, 497–500.

4. Hybridation des cellules L (HBs⁺) avec les hépatocytes en culture primaire.

Les hybridations ont été effectuées sur les hépatocytes après leur attachement dans les flacons Falcon, 6 heures ou 20 heures après la mise en culture. 10⁶ cellules L ont été ajoutées dans les cultures d'hépatocytes (2.5.10⁶ à 3.10⁶ cellules). Le processus de fusion est induit par un court contact avec le polyéthylène glycol, selon les modalités décrites par R.L. Davidson et al (1976), Somatic Cell Genetics Vol. 2, n° 2, 165–176. 24 à 28 heures plus tard, les cultures sont passées dans le milieu sélectif HAT (Littlefield 1964). Dans ces conditions, les cellules parentes de souris (cellules L TK-) sont éliminées. Il en est de même spontanément des hépatocytes fragilisés par la mise en culture et le contact avec le polyéthylène glycol. Les clones hybrides viables apparaissant dans le milieu de culture, sont isolés, mis en culture et testés quant à leur capacité de production de l'antigène HBs.

Des niveaux de production importants de l'antigène HBs (détecté et dosé comme indiqué ci-dessus) dans les clones hybrides se sont manifestés plus particulièrement dans les hybrides L1 × HA.

a) Sur 29 clones isolés:

– 2 ont été négatifs, c'est-à-dire n'ont pas produit d'HBsAG détectable,

– 27 ont eu une production supérieure aux cellules L1, selon la répartition suivante:

– 11 ont eu une production multipliée par 2 à 5 fois,

– 5 ont eu une production multipliée par 6 à 10 fois,

– 8 ont-eu une production multipliée par 10 à 20 fois,

– 2 ont eu une production multipliée par 20 à 40 fois,

– 1 ont eu une production multipliée par plus de 50 fois.

b) Sur 4 cultures comprenant les ensembles de clones non isolés, on a observé:

– pour l'une une production × 2,

– pour les 3 autres cultures, une production multipliée par un facteur compris entre 10 et 30.

Les hybrides obtenus à partir de L2 et L3, c'est-à-dire L2 × HA (8 clones isolés) et L3 × HA (12 clones isolés) n'ont pas produit d'HBs détectable.

Exemple II

On réalise une hybridation préalable entre les mêmes hépatocytes que ceux indiqués dans l'exemple I et des lignées cellulaires d'hépatome BWTG3 (HGPRT-) dans le milieu de HAT. L'hybridation est réalisée dans les mêmes conditions que celles décrites dans l'exemple I. Les hybrides cellulaires qui se sont développés dans ce milieu sont recueillis. La souche d'hybrides formée, utilisée dans la transformation complémentaire qui suit a été déposée à la C.N.C.M. le 5 mars 1984 sous le n° I-282.

Dans une deuxième étape, ladite lignée cellulaire hybride est alors cotransformée avec le plasmide pNY4 et le plasmide pW dans les conditions décrites par Garapin et al déjà mentionnées, dans un milieu de culture contenant l'antibiotique G418.

On recueille les cellules qui se sont développées dans ce milieu et qui, au surplus, témoignent d'une production de HBs révélable par le test immunologique mis en œuvre dans l'exemple précédent. On recueille ainsi des souches dont la production de HBs est du même ordre de grandeur que ceux indiqués dans l'exemple I.

En particulier on a isolé dans une première expérience 4 clones qui secrètent (pour 10⁶ cellules par 24 heures) respectivement: 800; 117; 20 et 625 ng de HBsAg et, dans une seconde expérience (toujours pour 10⁶ cellules par 24 heures) respectivement: 1781; 365; 548; 2810; 773; 863 ng de HBsAg.

Il est significatif que des essais de cotransformation réalisés dans des conditions semblables non pas sur l'hybride, mais sur la lignée cellulaire d'hépatome initiale n'ont pas conduit à des lignées secrétant des quantités plus que négligeables d'HBs.

Le plasmide pNY4 a été déposé à la C.N.C.M. le 5 mars 1984 sous le n° I-285 (dans E. coli K12).

On remarquera certes, au vu de l'exemple I, que certains clones d'hybrides se sont révélés ne pas exprimer la séquence d'ADN codant pour l'antigène HBs. Mais on remarquera en même temps que sur 3 clones prélevés au hasard, l'un d'entre eux s'est révélé secréter des proportions importantes d'HBs. Il est tout à fait naturel dans cette technique que l'on puisse, au hasard des essais, isoler un certain nombre de clones hybrides non opérationnels. L'exemple I montre cependant que le nombre d'essais de cultures à réaliser pour en sélectionner certains qui sont actifs, est suffisamment réduit pour être statistiquement reproductibles.

Dans les exemples qui précèdent, la séquence d'ADN déterminée était constituée pour l'essentiel par le gène S de DNA-HBV. Il va de soi que cette séquence aurait pu être remplacée par d'autres séquences, par exemple le gène codant pour l'antigène HBc ou pour tout autre gène ou séquence d'acide nucléique codant pour le même antigène. On remarquera aussi que la même méthode peut être étendue à l'utilisation de toute cellule primaire autorisant des expressions transitoires de séquences d'ADN déterminées ayant servi à leur transformation, à l'étude de l'expression de tout gène cloné postérieurement à l'hybridation des cellules de mammifère le contenant, avec le type cellulaire normal primaire différencié correspondant. D'une façon générale, la méthode selon l'invention peut être appliquée à l'obtention de tous clones hybrides hautement producteurs d'une protéine susceptible d'applications médicales ou industrielles, ou encore pour étudier les mécanismes qui contrôlent les gènes dans les cellules.

Comme il va de soi et comme il résulte d'ailleurs déjà de ce qui précède, l'invention ne se limite nullement à ceux de ses modes d'application et de réalisation qui ont été plus particulièrement envisagés; elle en embrasse, au contraire, toutes les variantes.

## Revendications

1. Procédé de fabrication d'un hybride cellulaire exprimant une séquence d'ADN clonée, caractérisée:
   - en ce que l'on réalise une coculture, dans des conditions propres à permettre leur hybridation, d'une part, de cellules eucaryotes établies, dans lesquelles la susdite séquence d'ADN clonée est exprimable ou exprimée et, d'autre part, de cellules primaires naturellement favorables à l'expression de gènes naturels codant pour une protéine constituée par le polypeptide codé par la susdite séquence d'ADN clonée ou contenant dans sa propre structure une séquence polypeptidique identique ou analogue à celle de ce polypeptide, cette coculture étant réalisée dans un milieu qui n'autorise pas le développement desdites cellules établies, lorsqu'elles ne sont pas complémentées par une séquence génétique appropriée,
   - en ce que les cellules primaires mises en jeu dans l'hybridation cellulaire sont aptes ou ont été rendues aptes à fournir ladite séquence génétique appropriée à au moins une partie des hybrides cellulaires formés dans ledit milieu de culture,
   - en ce que l'on sélectionne, après ladite coculture, ceux des hybrides cellulaires formés dans ledit milieu de culture,
   - et en ce que l'on recueille ces derniers hybrides cellulaires.

2. Procédé selon la revendication 1, caractérisé en ce que, soit les cellules établies, soit les cellule primaires, soit les deux à la fois, avaient été transformées, préalablement à la coculture susdite, avec la séquence d'ADN clonée susdite et en ce que l'on sélectionne, après ladite coculture, ceux des hybrides cellulaires formés, qui se révèlent aussi être producteurs du polypeptide codé par le susdit ADN cloné.

3. Procédé selon la revendication 1, caractérisé en ce que les hybrides cellulaires obtenus à l'issue de la susdite coculture sont eux-mêmes transformés avec ladite séquence d'ADN clonée et en ce que l'on sélectionne, après ladite coculture, ceux des hybrides cellulaires formés, qui se révèlent aussi être producteurs du polypeptide codé par le susdit ADN cloné.

4. Procédé selon l'une quelconque des revendications 1 à 3, caractérisé en ce que la séquence d'ADN clonée contient tout ou partie d'un gène déterminé contenu dans le génome du virus de l'hépatite B ou codant pour un peptide immunogène ayant des propriétés immunologiques équivalentes à celles du polypeptide codé par ce gène, et en ce que les cellules primaires mises en œuvre dans la coculture, consistent en des hépatocytes, de préférence humains.

5. Procédé selon l'une quelconque des revendications 1 à 4, caractérisé en ce que les cellules eucaryotes établies comportent une déficience au niveau de l'un de leurs gènes et que le milieu de culture est un milieu sélectif dans lequel lesdites cellules établies ne sont pas capables de se développer en raison de cette déficience.

6. Procédé selon la revendication 5, caractérisé en ce que les cellules eucaryotes sont des fibroblastes de souris L TK- ou HGPRT- et en ce que le milieu de culture est un milieu sélectif HAT.

7. Procédé selon la revendication 5, caractérisé en ce que les cellules eucaryotes établies sont constituées par des lignées d'hépatomes HGPRT- et en ce que le milieu de culture est constitué par un milieu sélectif HAT.

8. Procédé selon l'une quelconque des revendications 1 à 7, caractérisé en ce que le milieu de culture contient un antibiotique, notamment du type aminoglycoside, capable de pénétrer dans des cellules eucaryotes, et en ce que les cellules primaires, notamment les hépatocytes, ont au préalable été transformées avec une séquence d'ADN codant pour une enzyme capable d'inactiver l'antibiotique.

9. Procédé selon la revendication 7, caractérisé en ce que l'antibiotique est constitué par le G418 et en ce que la séquence d'ADN code pour une aminoglycoside 3'-phosphotransférase.

10. Procédé selon la revendication 4 prise seule ou en combinaison avec l'une quelconque des revendications 5 à 9, caractérisé en ce que la susdite séquence d'ADN clonée contient au moins une partie du gène S du virus de l'hépatite virale B et, de préférence aussi, le gène correspondant à la région pré-S du génome du virus de l'hépatite virale B.

11. Cellules hybrides obtenues par le procédé selon la revendication 10, caractérisées en ce qu'elles contiennent une partie du patrimoine génétique des hépatocytes primaires et en ce qu'elles sont capables de secréter un polypeptide immunogène présentant des propriétés vaccinantes à l'égard de l'hépatite virale B, avec un taux de production de polypeptide immunogène d'au moins 50 ng par $10^6$ cellules et par 24 heures.

**Patentansprüche**

1. Verfahren zur Herstellung eines Zellhybriden, der eine klonierte DNA-Sequenz exprimiert, dadurch gekennzeichnet, dass
– man unter Bedingungen, die geeignet sind, ihre Hybridisierung zu ermöglichen, eine Cokultur aus einerseits etablierten eukariotischen Zellen, in die die obengenannte klonierte DNA-Sequenz exprimierbar oder exprimiert ist, und andererseits Primärzellen, die von Natur aus zur Expression von natürlichen Genen begünstigt sind, welche ein Protein kodieren, das von dem Polypeptid gebildet wird, welches durch die obengenannte klonierte DNA-Sequenz kodiert wird oder in ihrer eigenen Struktur eine identische oder zu derjenigen des Polypeptids analoge Polypeptidsequenz enthalten, wobei diese Cokultur in einem Medium, das nicht die Entwicklung der etablierten Zellen zulässt, hergestellt worden ist, wenn sie nicht von einer geeigneten genetischen Sequenz komplementiert werden, herstellt,
– die bei der Zellhybridisierung verwendeten Primärzellen fähig sind oder fähig gemacht worden sind, diese geeignete genetische Sequenz an mindestens einen Teil der in diesem Kulturmedium gebildeten Zellhybride zu liefern,
– man nach dieser Cokultur diejenigen der Zellhybride, die in diesem Kulturmedium gebildet worden sind, auswählt und
– man die letzteren Zellhybride sammelt.

2. Verfahren nach Anspruch 1, dadurch gekennzeichnet, dass vor der obgenannten Cokultur die etablierten Zellen, die Primärzellen oder beide auf einmal mit der obengenannten klonierten DNA-Sequenz transformiert worden sind und man nach der Cokultur diejenigen der gebildeten Zellhybride, die sich ebenfalls als Produzenten des Polypeptids, welches von der obgenannten klonierten DNA kodiert wird, erweisen, auswählt.

3. Verfahren nach Anspruch 1, dadurch gekennzeichnet, dass man die nach Beendigung der obgenannten Cokultur erhaltenen Zellhybride selbst mit der klonierten DNA-Sequenz transformiert und nach der Cokultur diejenigen der gebildeten Zellhybride, die sich ebenfalls als Produzenten des Polypeptids, das durch die obgenannte klonierte DNA kodiert wird, erweisen, auswählt.

4. Verfahren nach einem der Ansprüche 1 bis 3, dadurch gekennzeichnet, dass die klonierte DNA-Sequenz ganz oder zum Teil ein bestimmtes Gen, das im Genom des Hepatitisvirus B enthalten ist oder für ein immunogenes Peptid kodiert, das immunologische Eigenschaften besitzt, die äquivalent zu denjenigen des durch dieses Gen kodierten Polypeptids sind, enthält und die in der Cokultur verwendeten Primärzellen aus vorzugsweise menschlichen Hepatozyten bestehen.

5. Verfahren nach einem der Ansprüche 1 bis 4, dadurch gekennzeichnet, dass die etablierten eukariotischen Zellen einen Mangel an einem ihrer Gene aufweisen und das Kulturmedium ein selektives Medium ist, in dem die etablierten Zellen nicht in der Lage sind, sich wegen dieses Mangels zu entwickeln.

6. Verfahren nach Anspruch 5, dadurch gekennzeichnet, dass die eukariotischen Zellen L TK-- oder HGPRT--Mausfibroplasten sind und das Kulturmedium ein selektives HAT-Medium ist.

7. Verfahren nach Anspruch 5, dadurch gekennzeichnet, dass die etablierten eukariotischen Zellen aus HGPRT--Hepatomstämmen gebildet sind und das Kulturmedium aus einem selektiven HAT-Medium gebildet ist.

8. Verfahren nach einem der Ansprüche 1 bis 7, dadurch gekennzeichnet, dass das Kulturmedium ein Antibiotikum, insbesondere des Aminoglykosidtyps, welches in die eukariotischen Zellen dringen kann, enthält, und die Primärzellen, insbesondere die Hepatozyten, zuvor mit einer DNA-Sequenz, die für ein Enzym kodiert, das das Antibiotikum inaktivieren kann, transformiert worden sind.

9. Verfahren nach Anspruch 7, dadurch gekennzeichnet, dass das Antibiotikum durch G418 gebildet wird und die DNA-Sequenz für eine Aminoglykosid-3'-Phosphotransferase kodiert.

10. Verfahren nach Anspruch 4 allein oder in Kombination mit einem der Ansprüche 5 bis 9, dadurch gekennzeichnet, dass die obengenannte klonierte DNA-Sequenz wenigstens einen Teil des S-Gens des Hepatitisvirus B und ebenfalls vorzugsweise das Gen, das der Pre-S-Region des Genoms des Hepatitisvirus B entspricht, enthält.

11. Zellhybride, erhalten durch das Verfahren nach Anspruch 10, dadurch gekennzeichnet, dass sie einen Teil des genetischen Vermögens der Primärhepatozyten enthalten und sie ein immunogenes Polypeptid, das Impfeigenschaften im Hinblick auf den Hepatitisvirus B vorweist, mit einer Produktion des immunogenen Polypeptids in einer Grössenordnung von wenigstens 50 ng pro 10⁶ Zellen während 24 Stunden ausscheiden können.

## Claims

1. A process for the manufacture of a cellular hybrid expressing or susceptible of being rendered capable of expressing a cloned sequence of DNA, characterized:
- in that one realizes a co-culture, under conditions apt to permit their hybridization, on the one hand, of established eucaryotic cells, in which the aforesaid cloned sequence of DNA is expressible or expressed and, on the other hand, of primary cells naturally favorable to the expression of natural genes coding for a protein constituted of the polypeptide coded by the aforesaid cloned DNA sequence or containing in it's own structure a polypeptide sequence identical or analog to that of this polypeptide, this co-culture being realized in a medium which does not allow the development of the said established cells, when they are not complemented by an appropriate genetic sequence,
in that the primary cells used in the cellular hybridization are apt or have previously been rendered apt to provide the said appropriate genetic sequence to at least a part of the cellular hybrids formed in the said culture medium and in that one selects the cellular hybrids formed in said culture-medium.

2. The process according to Claim 1, characterized in that, either the established cells, or the primary cells, or both at the same time, have been transformed, beforehand in the aforesaid co-culture, with the aforesaid cloned DNA sequence, and in that one selects, after the said co-culture, those of the cellular hybrids formed, which prove thus to be producers of the polypeptide coded by the said cloned DNA.

3. The process according to Claim 1, characterized in that the cellular hybrids obtained at the issue of the aforesaid co-culture are themselves transformed with the said cloned DNA sequence and in that one selects, after the said co-culture, those of the cellular hybrids formed, which show themselves also to be producers of the polypeptide coded by the aforesaid cloned DNA.

4. Process according to anyone of claims 1 to 3, characterized in that the cloned DNA sequence contains all or part of a determined gene contained in the genome of the hepatitis B virus or coding for an immunogenic peptide having the immunologic properties equivalent to those of the polypeptide coded by this gene, and in that said primary cells used in said co-culture consist of hepatocytes, preferably human ones.

5. Process according to anyone of claims 1 to 4, characterized in that the established eucaryotic cells have a deficiency at the level of one of their genes and that the culture medium is a selective medium in which the said established cells are not capable of developing because of this deficiency.

6. Process according to claim 5, characterized in that the eucaryotic cells are fibroblasts of L TK⁻ or HGPRT⁻ mice and in that the culture medium is a HAT selective medium.

7. Process according to claim 5, characterized in that the established eucaryotic cells are constituted by the HGPRT⁻ hepatoma lines and in that the culture medium is constituted of a HAT selective medium.

8. Process according to any one of the claims 1 to 7, characterized in that the culture medium contains an antibiotic, notably to the aminoglycoside type, capable of penetrating into eucaryotic cells and in that the primary cells particularly the hepatocytes, have been previously transformed with a sequence of DNA coding for an enzyme capable of inactivating the antibiotic.

9. Process according to claim 7, characterized in that the antibiotic is constituted of G418 and in that the DNA sequence codes for an aminoglycoside 3'-phosphotransferase.

10. Process according to claim 4 taken alone or in combination with any one of the claims 5 to 9, characterized in that the aforesaid cloned DNA contains at least a part of the S gene of the virus of viral hepatitis B and, preferably also, the gene corresponding to the pre-S region of the genome of the virus of viral hepatitis B.

11. Hybrid cells obtained by the process according to claim 10, characterized in that they contain a part of the genetic inheritance of the primary hepatocytes, and in that they are capable of secreting an immunogenic polypeptide presenting vaccinating properties with regard to viral hepatitis B, with a rate of production of immunogenic polypeptide of at least 50 ng per 10⁶ cells and per 24 hours.